# EUROPEAN PATENT APPLICATION

(11) **EP 4 850 645 A1**
(43) Date of publication of application: **09.12.2026**
(21) Application number: 25713372.8
(22) Date of filing: 21.02.2025
(51) Int. Cl.: A61F 13/01, A61F 5/30, A61F 13/14

(54) **PAD FOR POST-SURGERY TREATMENT**

(30) Priority: 29.02.2024 ES 202430402 U
(71) Applicant: Define Foam, S.L., 46018 Valencia (ES)
(72) Inventor: ALBERT ALBIR, Alejandro, 46018 Valencia Valencia (ES); BAYARRI ALMENAR, Raquel, 46018 Valencia Valencia (ES)
(74) Representative: Sahuquillo Huerta, Jesús
(86) International application number: PCT/ES2025/070089
(87) International publication number: WO 2025/181409

(57) **Abstract**

Pad for post-surgery treatment consisting of a body made of a hypoallergenic material and covered with silicone gel on one side, comprising a plurality of reliefs configured to orientate the pressure areas on the user's body and characterised in that it has a first relief area (1) in the shape of an inverted triangle with the lower vertex sectioned, a second relief area (2) and a third relief area (3) in the shape of a rectangle bisected by a circle at its upper and lower ends and at one of its sides, and a fourth relief area (4) and a fifth relief area (5) in the shape of a right-angled triangle, bisected by a circle at one of its vertices, connected to an assembly consisting of an arc and a line segment.

## Description

### Object of the invention

The object of the present report is a pad for post-surgery treatment in interventions such as liposuction or abdominoplasty, lipo-marcation or any intervention that requires the support of this abdominal or lumbar area, The pad is made of a hypoallergenic material such as polyurethane or other material with equivalent characteristics, which is covered by a platinum silicone-based gel cover or layer with a hardness of less than 0 shores on one of its sides and incorporates a plurality of reliefs, which facilitate the reduction of fibrosis production and the non-accumulation of seroma in the lipo-marking areas thanks to the pressure exerted on the areas with reliefs.

### Background to the invention

In the world of cosmetic surgery, liposuction has become one of the most common and sought-after cosmetic procedures. The procedure is designed to remove unwanted fat deposits from specific areas of the body by suctioning fat using thin cannulas inserted through small incisions in the skin. This procedure is not intended for general weight loss, but rather to shape and contour specific areas of the body.

Liposuction is usually performed in areas where fat accumulates persistently and is resistant to diet and exercise. Some of the common areas for liposuction include abdomen, thighs, buttocks, flanks (the sides of the torso), back, arms, etc.

Once the procedure has been performed, the postoperative period after liposuction is crucial for a successful recovery and to obtain the desired results. In this process it is essential to rest and allow the body to recover, as well as to stay well hydrated and follow a healthy diet. In addition, the use of compression garments is often recommended postoperatively to provide the compression that the skin and muscles need and to help the affected tissue adapt to its new shape, as well as to ensure clean incisions and prevent infection, reduce swelling, minimise bruising and maintain the desired shape. These garments are usually required for several weeks after the procedure

At present, the applicant is not aware of a pad for the treatment of liposuctions made of a hypoallergenic material with a part covered by a platinum silicone-based gel of a medical nature; and which presents a plurality of reliefs that facilitate the reduction of fibrosis production and the non-accumulation of seroma thanks to its pressure in the pronounced areas as the one proposed in the present descriptive report.

### Description of the invention

The technical problem solved by the present invention is to facilitate the reduction of fibrosis production and the non-accumulation of seroma in the lipo-marcation areas. For this purpose, the pad for post-surgery treatment, object of the present invention, will be made of a hypoallergenic material such as polyurethane or equivalent and covered by a platinum silicone-based gel on one of its sides, presenting a plurality of reliefs configured to orientate the areas of pressure on the user's body.

This pad can be used immediately after surgery and is placed between the treated area and a compression garment, helping to better distribute the compression thanks to the plurality of reliefs it has.

Thanks to its materialisation in a hypoallergenic material, the pad has a series of advantages that facilitate its use and its implantation in different types of treatment such as liposuction, such as: it improves skin adherence, provides comfort, reduces irritation and the production of haematomas as well as providing adequate compression in the treatment area, helping recovery and reducing inflammation in the operated area.

In addition to the use of this material, the cushion has one of its sides covered with platinum silicone gel with a hardness of less than 0 shores, which makes it a resistant material without losing its properties of adaptability and lightness.

Throughout the description and the claims, the word "comprises" and its variants are not intended to exclude other technical features, additives, components or steps. To those skilled in the art, other objects, advantages and features of the invention will be apparent in part from the description and in part from the practice of the invention. The following examples and drawings are provided by way of illustration and are not intended to restrict the present invention. Furthermore, the present invention covers all possible combinations of particular and preferred embodiments indicated herein.

### Brief description of the figures

The following is a very brief description of a drawing which helps to better understand the invention, and which relates expressly to an embodiment of the invention which is presented as a non-limiting example of the invention.

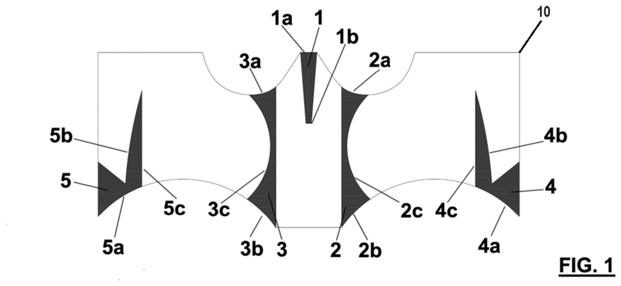

FIG 1. It shows a view of the pad for post-surgery treatment, object of the invention.

### Detailed description of a detailed embodiment of the invention

The attached figure shows a preferred embodiment of the invention. More specifically, the pad for post-surgery treatment comprises a body made of a hypoallergenic material such as polyurethane or equivalent and covered by a platinum silicone-based gel on one of its sides, with several reliefs configured to orientate the pressure areas on the user's body
In a preferred embodiment, the pad (10) comprises a width of between 70 and 95 centimetres, a height of between 30 and 40 centimetres and a thickness of between 1 and 2 centimetres. Likewise, the plurality of reliefs comprising the pad has an extra thickness of between 0.5 and 1.5 centimetres.

The pad (10) has a first relief area (1) in the shape of an inverted triangle with a sectioned lower vertex, comprising an upper base (1a) of between 2 and 4 centimetres and a lower base (1b) of between 0.5 and 1.5 centimetres.

In turn, it has a second relief area (2) and a third relief area (3) in the shape of a rectangle sectioned by a circle at its upper and lower ends as well as at one of its sides, comprising an upper section (2a, 3a) with a radius of between 4 and 6 centimetres, a lower section (2b, 3b) with a radius of between 19 and 21 centimetres and a side section (2c, 3c) with a radius of between 12 and 14 centimetres and a side section (2d, 3d) with a length of between 24 and 26 centimetres.

Finally, it has a fourth relief area (4) and a fifth relief area (5) in the shape of a right-angled triangle, bisected by a circle at one of its vertices, connected to an assembly consisting of an arc and a line segment, comprising a circular section (4a, 5a) of between 19 and 21 centimetres in radius, an arc (4b, 5b) of between 78 and 82 centimetres in radius, and a side (4c, 5c) of between 8 and 12 centimetres in length.

In a particular embodiment, the first relief area (1) comprises an upper base (1a) of 3 centimetres as a preferred size and a lower base (1b) of between 1 centimetre as a preferred size.

## Claims

1. Pad for post-surgery treatment consisting of a body made of a hypoallergenic material and covered with silicone gel on one side, comprising a plurality of reliefs configured to orientate the pressure zones on the user's body and **characterised in that** it has a first relief zone (1) in the shape of an inverted triangle with the lower vertex sectioned, a second relief area (2) and a third relief area (3) in the shape of a rectangle bisected by a circle at its upper and lower ends and at one of its sides, and a fourth relief area (4) and a fifth relief area (5) in the shape of a right-angled triangle, bisected by a circle at one of its vertices, connected to an assembly consisting of an arc and a line segment.

2. Pad for post-surgery treatment according to claim 1 wherein the first relief area (1) comprises an upper base (1a) of between 2 and 4 centimetres and a lower base (1b) of between 0.5 and 1.5 centimetres.

3. Pad for post-surgery treatment according to claim 2 wherein the first relief area (1) comprises an upper base (1) of 3 centimetres as preferred size and a lower base (1b) of 1 centimetre as preferred size.

4. Pad for post-surgery treatment according to claim 1 wherein the second relief area (2) and the third relief area (3) comprise an upper section (2a, 3a) of between 4 and 6 centimetres in radius, a lower section (2b, 3b) of between 19 and 21 centimetres in radius and a side section (2c, 3c) of between 12 and 14 centimetres in radius, as well as a side (2d, 3d) of between 24 and 26 centimetres in length.

5. Pad for post-surgery treatment according to claim 1 wherein the fourth relief area (4) and the fifth relief area (5) comprises a circular section (4a, 5a) with a radius of between 19 and 21 centimetres, an arc (4b, 5b) with a radius of between 78 and 82 centimetres, as well as a side (4c, 5c) with a length of between 8 and 12 centimetres.

6. Pad for post-surgery treatment according to claim 1 wherein the pad (10) comprises a width of between 70 and 95 centimetres, a height of between 30 and 40 centimetres as well as a thickness of between 1 and 2 centimetres.

7. Pad for post-surgery treatment according to claim 1 wherein the first relief area (1), the second relief area (2), the third relief area (3), the fourth relief area (4) and the fifth relief area (5) comprise an extra thickness of between 0.5 and 1.5 centimetres.

8. Pad for post-surgery treatment according to any one of the preceding claims wherein the body forming the pad is made of hypoallergenic polyurethane.
